# FASCICULE DE BREVET EUROPEEN

(11) **EP 1 627 624 B1**
(45) Date de publication et mention de la délivrance du brevet: **18.02.2009**
(21) Numéro de dépôt: 05291594.9
(22) Date de dépôt: 26.07.2005
(51) Int. Cl.: A61K 8/18, A61K 8/04, A61K 8/81, A61K 8/88, A61Q 19/00

(54) **Composition topique comprenant des particules poreuses chargées et un composé absorbant le sébum**
Topische Zusammensetzung enthaltend poröse Partikel und eine Sebum absorbierende Verbindung
Topical composition containing loaded porous particles and a sebum absorbing compound

(30) Priorité: 17.08.2004 FR 0408937
(43) Date de publication de la demande: 22.02.2006
(73) Titulaire: L'ORÉAL, 75008 Paris (FR)
(72) Inventeur: Josse, Delphine, 94250 Gentilly (FR)
(74) Mandataire: Kromer, Christophe

(56) Documents cités:
- EP-A- 0 717 979
- EP-A- 1 493 433
- WO-A-96/17583
- WO-A-20/04075872
- WO-A-20/05011622
- FR-A- 2 657 255
- FR-A- 2 857 254
- US-A1- 2003 032 680

## Description

La présente invention se rapporte à un procédé cosmétique de soin des peaux grasses, comprenant l'application topique sur la peau une composition comprenant, dans un milieu physiologiquement acceptable adapté à une application topique sur la peau et renfermant une phase aqueuse : (a) des particules poreuses ayant un diamètre moyen, en volume, inférieur ou égal à 10 µm et renfermant au moins un actif de soin des peaux grasses, et (b) au moins un composé absorbant et/ou adsorbant le sébum, ayant une taille moyenne de particule en nombre supérieure à 10 µm.

On connaît de la demande EP-1 493 433 des compositions cosmétiques comprenant des particules poreuses chargées d'actifs cosmétiques ou dermatologiques, destinés notamment au soin des peaux grasses. Ces particules ont des dimensions suffisamment petites pour faire pénétrer l'actif dans les pores de la peau et le transporter ainsi sur son site d'action, dans l'unité pilo-sébacée. L'Exemple 1 divulgue ainsi une poudre matifiante comprenant de l'acide salicylique adsorbé sur des particules de silice, ainsi que du talc à hauteur de 69% en poids.

On connaît par ailleurs de la demande FR 04/50887 (non publiée) des compositions matifiantes comprenant des composés absorbant ou adsorbant le sébum, associés à des charges à effet optique telles que des microsphères de polyamide (Nylon) ayant une taille moyenne égale à 10 µm. Il n'est pas suggéré dans cette demande que les microsphères de Nylon puissent être chargées d'un actif.

Il subsiste le besoin de disposer de compositions permettant de lutter plus efficacement contre les imperfections cutanées des peaux grasses que celles proposées à ce jour dans l'art antérieur.

La Demanderesse a découvert que ce besoin pouvait être satisfait en associant aux particules poreuses chargées précitées des composés susceptibles d'absorber ou d'adsorber le sébum, dans une composition contenant une phase aqueuse. La composition ainsi obtenue permet d'améliorer la matité de la peau de façon rémanente. En effet, les particules poreuses chargées d'actif, en désincrustant le canal pilo-sébacé, induisent dans un premier temps une augmentation de la production de sébum à la surface de la peau, qui peut être capté par les charges absorbant ou adsorbant le sébum. La peau reste ainsi durablement matifiée.

On connaît certes des demandes WO 96/17585 et FR-2 657 255 des compositions cosmétiques anhydres contenant des particules de silice, éventuellement enrobées, sur ou dans lesquelles sont adsorbés des actifs et qui peuvent être associées à des particules exerçant la fonction de pompes à sébum telles que le talc ou des agglomérats de copolymère réticulé (POLYTRAP®). Dans ces documents, les exemples de compositions contenant ces deux types de particules ne contiennent pas d'actif de soin des peaux grasses.

On connaît en outre de la demande US 2003/032680 une émulsion H/E renfermant de l'hydroquinone imprégnée sur des microparticules poreuses et absorbée dans des micro-agglomérats de plus de 10 µm (POLYTRAP®). Cette émulsion peut contenir divers actifs - dont les anti-oxydants - associés à l'hydroquinone. Cette émulsion n'est pas destinée au soin des peaux grasses mais plutôt au blanchiment de la peau.

On connaît aussi de la demande FR-2 857 254 des compositions dans lesquelles un azurant optique est imprégné dans des particules minérales poreuses de moins de 50 µm, de préférence de moins de 10 µm. Cette composition peut être destinée au soin des peaux grasses et contenir une pompe à sébum. Il n'est pas prévu que les particules puissent être imprégnées d'un actif de soin des peaux grasses.

On connaît en outre de la demande WO 05/011622 des particules poreuses à base de silice non sphérique, sur lesquelles est adsorbée une substance optiquement active, et qui peuvent être associées à une poudre sphérique de 0,2 à 20 µm telle que le Nylon. Là encore, il n'est pas prévu que les particules puissent être imprégnées d'un actif de soin des peaux grasses.

La présente invention a donc pour objet procédé cosmétique de soin des peaux grasses, comprenant l'application topique sur la peau d'une composition comprenant, dans un milieu physiologiquement acceptable adapté à une application topique sur la peau et renfermant une phase aqueuse : (a) des particules poreuses ayant un diamètre moyen, en volume, inférieur ou égal à 10 µm et renfermant au moins un actif de soin des peaux grasses, et (b) au moins un composé absorbant et/ou adsorbant le sébum ayant une taille moyenne de particule en nombre supérieure à 10 µm.

Les constituants de la composition selon l'invention seront maintenant décrits plus en détail.

### Particules poreuses

Par l'expression « particules poreuses », on désigne des particules ayant une structure comportant des pores. Cette structure poreuse peut notamment permettre au moins en partie l'incorporation d'un ou plusieurs agents actifs au sein desdites particules.

Cette structure peut être de type matriciel à l'image d'une éponge. Elle peut également être de type vésiculaire, c'est-à-dire que la particule présente une cavité interne délimitée par une paroi poreuse.

La porosité associée à la taille des particules est caractérisée quantitativement par leur surface spécifique. Les particules poreuses de l'invention présentent une surface spécifique mesurée selon la méthode BET supérieure ou égale à 1 m²/g.

La méthode BET (BRUNAUER-EMMET-TELLER) est une méthode bien connue de l'homme du métier. Elle est notamment décrite dans « The journal of the American Chemical Society », vol.60, page 309, février 1938 et correspond à la norme internationale ISO 5794/1 (annexe D). La surface spécifique mesurée selon la méthode BET correspond à la surface spécifique totale, c'est-à-dire qu'elle inclue la surface formée par les pores.

Selon un mode de réalisation particulier, les particules de l'invention présentent une surface spécifique mesurée par BET, allant notamment de 2,5 à 1000 m²/g, en particulier de 3 à 750 m²/g, plus particulièrement supérieure ou égale à 300 m²/g, voire supérieure ou égale à 500 m²/g.

Les particules poreuses selon l'invention sont généralement des particules individualisées. Par l'expression « particules individualisées », on désigne des particules qui ne sont pas regroupées sous la forme d'agrégat ou d'agglomérat. Ces particules présentent en particulier une masse volumique supérieure ou égale à 0,15 g/cm³ et notamment allant de 0,2 à 5 g/cm³.

Les particules utilisées selon la présente invention dérivent de particules poreuses préformées, c'est-à-dire formées en l'absence du ou des composé(s) à encapsuler.

Au sens de la présente invention, on utilisera ci-après l'expression « particules chargées » pour désigner les particules selon l'invention de manière à les distinguer du matériau particulaire dont elles dérivent et qui ne contient pas de composé actif.

Comme mentionné précédemment, les particules selon la présente invention ont un diamètre moyen en volume inférieur ou égal à 10 µm. En effet, de telles particules peuvent pénétrer dans le follicule sébacé par application d'une force mécanique. Cette force mécanique provient généralement d'un massage qui, outre la poussée qu'elle exerce, génère un effet de pompe au niveau du follicule. Les particules atteignent ainsi progressivement le canal folliculaire dans lequel le composé actif qu'elles portent peut alors diffuser, et accéder éventuellement aux tissus environnant le canal folliculaire. En revanche, le support, qui constitue la particule, est ensuite rejeté grâce à l'écoulement de sébum et/ou à la pousse du poil permettant ainsi d'éviter toute réaction indésirable de l'organisme vis-à-vis du composé solide constituant les particules.

II faut noter que des particules ayant un diamètre supérieur à 10 µm, même avec application d'une force mécanique similaire, restent pour la plupart localisées sur la surface de la peau sans y pénétrer et ne peuvent donc relarguer le composé actif que sur la couche cornée.

Selon un mode de réalisation particulier de l'invention, les particules ont un diamètre moyen en volume supérieur ou égal à 0,1 µm et en particulier allant de 0,5 à 8 µm.

Les particules utilisées selon l'invention sont des particules, notamment sphériques, poreuses, de dimension moyenne en nombre pouvant aller de 0,1 à 50 µm, notamment de 0,1 à 20 µm et tout particulièrement de 0,5 à 10 µm.

Selon une variante de l'invention, les particules se caractérisent par leur homogénéité granulométrique. En particulier, elles présentent un indice de polydispersité, IP, allant de 1 à 4, et notamment inférieur ou égal à 3. Cet indice de polydispersité est défini comme le rapport D(4,3)/D(3,2), dans lequel D(4,3) désigne le diamètre moyen en volume et D(3,2) désigne le diamètre moyen en surface. Ces deux valeurs sont communément mesurées à l'aide de granulomètres à diffraction laser tel que celui commercialisé sous la dénomination de « Mastersizer 2000 » par la société MALVERN.

Les particules poreuses de l'invention peuvent avoir des formes variées, notamment globulaire et en particulier sensiblement sphérique.

Ces particules poreuses sont généralement constituées de matériaux totalement insensibles, en termes notamment de solubilisation et de plastification, au procédé d'encapsulation des composés actifs, en particulier dans le cas où celui-ci fait intervenir pour l'imprégnation un solvant organique.

Ces particules peuvent être de type organique, inorganique ou mixte et se présentent le plus souvent sous forme de poudre ayant en particulier une volatilité réduite.

Comme particules poreuses de type organique, on citera à titre d'exemple les particules de polyamide, en particulier de Nylon 6, Nylon 6-6, Nylon 12 ou Nylon 6-12 telles que celles commercialisées par la société ATOFINA sous le nom générique d'« Orgasol », et les particules de polyméthacrylate de méthyle (PMMA) telles que celles commercialisées sous la dénomination de « Covabead® » par la société WAKER.

Dans un mode de réalisation particulier de l'invention, les particules utilisées sont choisies parmi les particules de polyamide (Nylon®) telles que celles citées ci-dessus.

Selon une variante de l'invention, les particules selon l'invention sont de nature inorganique.

A titre illustratif des matériaux inorganiques utilisables dans les particules selon l'invention, on peut citer la silice, les composites silice-alumine, l'hydroxyapatite, le dioxyde de titane, la séricite, le mica, le carbonate ou l'hydrocarbonate de magnésium, les oxydes d'aluminium de type alumine et les silicates mixtes, comme les aluminosilicates, et leurs mélanges.

En particulier, les particules inorganiques poreuses convenant à l'invention peuvent être choisies parmi :
- les particules de silice comme celles commercialisées par ASAHI GLASS sous le nom de « Sunsphere H series », et par SUZUKI OIL AND FAT sous le nom de « God Balls »,
- les particules d'hydroxyapatite comme celles commercialisées par MERCK (sous la référence 1051990010 - granulométrie 15 µm), ou bien celles commercialisées par les sociétés LABORATORY SKIN CARE et SEKISUI sous les noms respectifs « Hydroxyzomes » (LSC), AP20C et AP12C (SEKISUI), d'« ASP® » par la société SEKISUI PLASTICS ou la dénomination d'«Hydrozyzomes » par la société ASAHI GLASS
- les particules de silice-alumine telles que celles commercialisées sous la dénomination de « Zeeosphere® » par la société 3M,
- les particules de dioxyde de titane telles que celles commercialisées par la société ISHIHARA, et
- les particules composées de mélange de ces minéraux.

Dans un mode de réalisation de l'invention, les particules utilisées sont en particulier choisies parmi des particules de silice et d'hydroxyapatite.

Les particules poreuses utilisées dans la présente invention peuvent être également constituées de matériaux composites organiques et inorganiques.

Les particules chargées de la présente invention sont préparées selon des méthodes conventionnelles, en particulier par imprégnation. En particulier, les particules chargées selon l'invention sont obtenues par imprégnation de particules poreuses préformées avec au moins un composé actif. Avantageusement, ce protocole ne requiert pas la présence d'un porogène.

A titre d'exemple, les particules poreuses renfermant l'actif sont susceptibles d'être obtenues selon un procédé d'imprégnation comprenant les étapes suivantes :
- la solubilisation de l'actif à encapsuler dans un solvant tel que l'acétone ou l'éthanol, pour obtenir une solution d'imprégnation,
- l'imprégnation des particules poreuses par ladite solution d'imprégnation,
- l'évaporation du solvant jusqu'à l'obtention d'une poudre sèche.

La poudre ainsi obtenue ne contient généralement qu'une très faible proportion de solvant résiduel, de l'ordre de 1/10 ppm.

Comme solvants pouvant être utilisés dans un tel procédé d'imprégnation, on peut citer notamment l'acétone, l'éthanol, l'isopropanol, le dichlorométhane, l'acétate d'éthyle. Bien entendu, le choix du solvant est effectué en tenant compte de la nature des composants des particules poreuses et des composés à encapsuler.

Généralement, la composition contient de 0,1 à 50 % en poids, de préférence de 0,2 à 20 % en poids, et plus préférentiellement de 1 à 5% en poids de particules poreuses chargées, par rapport au poids total de la composition.

### Actif de soin des peaux grasses

Par l'expression « actif de soin des peaux grasses », on entend, dans le cadre de la présente invention, un composé qui a par lui-même, c'est-à-dire ne nécessitant pas l'intervention d'un agent extérieur pour l'activer, une activité biologique qui peut être en particulier :
- une activité desquamante (qui permet l'ouverture des comédons), et/ou
- une activité anti-microbienne (notamment sur *P. acnes*), et/ou
- une activité anti-inflammatoire, et/ou
- une activité anti-oxydante (qui empêche l'oxydation du squalène et la formation des comédons).

L'actif de soin des peaux grasses peut donc être choisi parmi : les agents desquamants et/ou anti-microbiens et/ou anti-inflammatoires et/ou anti-oxydants.

### 1. Agents desquamants

Par "agent desquamant", on entend tout composé capable d'agir directement sur la desquamation en favorisant l'exfoliation choisi parmi l'acide n-octanoyl 5-salicylique et l'acide cinnamique.

L'acide n-octanoyl-5-salicylique est préféré pour une utilisation dans la présente invention.

### 2. Agents anti-microbiens

Les agents anti-microbiens susceptibles d'être utilisés dans la composition selon l'invention sont choisis parmi le 2,4,4'-trichloro-2'-hydroxy diphényl éther (ou triclosan), l'acide undécylenique, l'acide lipoïque, l'acide azélaïque, l'iodopropynyl butylcarbamate et le farnesol.

L'agent antimicrobien préféré est l'iodopropynyl butylcarbamate.

### 3. Agents anti-inflammatoires

Comme agents anti-inflammatoires ou apaisants utilisables dans la composition selon l'invention, on peut citer : l'acide β-glycyrrhétinique, le stearyl glycyrrhetinate, l'acide 3- stéaroyloxy glycyrrhetique), l'acide ursolique et le bisabolol.

### 4. Agents anti-oxydants

Les agents anti-oxydants préférés pour une utilisation dans la présente invention sont choisis parmi le tocophérol et l'acétate de tocophérol.

Le ou les actifs utilisés dans la composition selon l'invention peuvent représenter de 1 à 50%, de préférence de 2 à 40% et, mieux, de 5 à 30% du poids total des particules chargées.

### Composé absorbant et/ou adsorbant le sébum

On entend par « composé absorbant le sébum », un composé apte à absorber et/ou adsorber le sébum, couramment désigné par "pompe à sébum".

Généralement, ce type de composé se présente sous la forme d'une poudre de particules ayant une prise de sébum. De manière avantageuse, la prise de sébum de ces composés est supérieure ou égale à 1 ml/g, notamment supérieure ou égale à 2 ml/g, et en particulier supérieure ou égale à 3 ml/g. La prise de sébum correspond à la quantité de sébum absorbée et/ou adsorbée sur la surface disponible des particules.

Elle est mesurée selon la méthode dite de Wet Point ou méthode de détermination de prise d'huile de poudre décrite dans la norme NF T 30-022. Elle correspond à la quantité de sébum adsorbée sur la surface disponible de la poudre et/ou absorbée par la poudre par mesure du Wet Point, décrite ci-dessous :

On place une quantité m (en grammes) de poudre comprise entre environ 0,5 g et 5 g (la quantité dépend de la densité de la poudre) sur une plaque de verre puis on ajoute goutte à goutte du sébum artificiel, maintenu à la température de 29 °C et ayant la composition suivante exprimé en % en poids:
- trioléine 29,00 %
- acide oléïque 28,50 %
- oléate d'oléyle 18,50 %
- squalène 14,00 %
- cholestérol 7,00 %
- palmitate de cholestérol 3,00 %

Après addition de 4 à 5 gouttes de sébum artificiel, on incorpore le sébum artificiel dans la poudre à l'aide d'une spatule et on continue d'ajouter du sébum artificiel jusqu'à la formation de conglomérats de sébum artificiel et de poudre. A partir de ce moment, on ajoute le sébum artificiel à raison d'une goutte à la fois et on triture ensuite le mélange avec la spatule. On cesse l'addition de sébum artificiel lorsque l'on obtient une pâte ferme et lisse. Cette pâte doit se laisser étendre sur la plaque de verre sans craquelures ni formation de grumeaux. On note alors le volume Vs (exprimé en ml) de sébum artificiel utilisé.

La prise de sébum correspond au rapport Vs / m.

Les particules de composé absorbant le sébum doivent présenter une taille moyenne supérieure à 10 µm, pour ne pas entrer en compétition avec les particules poreuses décrites précédemment.

Selon un mode de réalisation particulier, les particules de composé absorbant et/ou adsorbant le sébum peuvent présenter une surface spécifique BET supérieure ou égale à 300 m²/g, notamment supérieure ou égale à 500 m²/g, et en particulier supérieure ou égale à 600 m²/g, et notamment inférieure ou égale à 1500 m²/g.

Les particules de composé absorbant et/ou adsorbant le sébum peuvent être d'origine minérale ou organique, plus particulièrement d'origine organique. Plus précisément, ce composé peut être choisi parmi les poudres de polyamide (Nylon®), les poudres de polymères acryliques, notamment de polyméthacrylate de méthyle/diméthacrylate d'éthylène glycol, de polyméthacrylate d'allyle/diméthacrylate d'éthylène glycol et de copolymère diméthacrylate d'éthylène glycol/méthacrylate de lauryle, les poudres de polyalkylstyrène réticulé, et leurs mélanges.

Les particules de ce composé peuvent, le cas échéant, être traitées en surface par au moins un agent de traitement hydrophobe, notamment non fluoré.

Cet agent de traitement hydrophobe peut notamment être choisi parmi:
- les silicones, comme les méthicones, les diméthicones ;
- les acides gras, comme l'acide stéarique ;
- les savons métalliques, comme le dimyristate d'aluminium, le sel d'aluminium du glutamate de suif hydrogéné ;
- les acides aminés, les acides aminés N-acylés ou leurs sels;
- la lécithine, le triisostéaryle titanate d'isopropyle ; et
- leurs mélanges.

Les acides aminés N-acylés peuvent comprendre un groupe acyle comprenant de 8 à 22 atomes de carbone, comme par exemple un groupe 2-éthyl hexanoyle, caproyle, lauroyle, myristoyle, palmitoyle, stéaroyle et cocoyle. Les sels de ces composés peuvent être les sels d'aluminium, de magnésium, de calcium, de zirconium, de zinc, de sodium ou de potassium. L'acide aminé peut être par exemple la lysine, l'acide glutamique ou l'alanine.

A titre représentatif et non limitatif des composés absorbant et/ou adsorbant le sébum selon l'invention, on peut tout particulièrement citer :
- les poudres de polyamides (Nylon®), comme par exemple "l'ORGASOL® 2002 D NAT COS" commercialisé par la société ATOFINA,
- les poudres de polymères acryliques, notamment de polyméthacrylate de méthyle/diméthacrylate d'éthylène glycol, comme par exemple le "DOW CORNING 5640 MICROSPONGE® SKIN OIL ADSORBER" commercialisé par la société DOW CORNING ou "MICROPEARL M 305" commercialisé par la société MATSUMOTO YUSHI ; de polyméthacrylate d'allyle/diméthacrylate d'éthylène glycol, comme par exemple le "POLY-PORE® E200" commercialisé par la société AMCOL ; de copolymère diméthacrylate d'éthylène glycol/méthacrylate de lauryle, comme par exemple le "POLYTRAP® 6603" commercialisé de la société RP SCHERER, et
- les poudres de polyalkylstyrène réticulé telles que les "IMBIBER BEADS 295" commercialisées par la société IMBIBITIVE TECHNOLOGIES.

Le composé absorbant ou adsorbant le sébum peut représenter de 0,1 à 10% en poids, et de préférence de 1 à 5% en poids, par rapport au poids total de la composition.

La composition selon l'invention est généralement adaptée à une application topique sur la peau et comprend donc généralement un milieu physiologiquement acceptable, c'est-à-dire compatible avec la peau et/ou ses phanères. Il s'agit de préférence d'un milieu cosmétiquement acceptable, c'est-à-dire qui présente une couleur, une odeur et un toucher agréables et ne génère pas d'inconforts inacceptables (picotements, tiraillements, rougeurs), susceptible de détourner la consommatrice d'utiliser cette composition.

La composition selon l'invention peut se présenter sous toutes les formes galéniques classiquement utilisées pour une application topique et notamment sous forme de dispersions du type lotion ou gel, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème ou gel, ou encore d'émulsions multiples (E/H/E ou H/E/H), de microémulsions, de dispersions vésiculaires de type ionique et/ou non ionique, ou de dispersions cire/phase aqueuse. Ces compositions sont préparées selon les méthodes usuelles.

Selon un mode préféré de réalisation de l'invention, la composition se présente sous forme d'une émulsion H/E.

La composition selon l'invention peut être une composition pour le soin, le nettoyage ou le maquillage de la peau du corps ou du visage

Cette composition peut en outre contenir divers adjuvants couramment utilisés dans le domaine cosmétique, tels que des émulsionnants dont les esters d'acide gras et de glycéryle, les esters d'acide gras et de sucre éventuellement polyoxyéthylénés, les esters d'acide gras et de polyoxyéthylène, les esters d'acide gras et de sorbitane ; des charges, notamment des charges à effet soft-focus telles que des dispersions colloïdales de silice ou de charge composite silice-alumine ou encore des fibres de polyamide ; des conservateurs ; des séquestrants ; des colorants ; des parfums ; et des épaississants et gélifiants, en particulier les homo- et copolymères d'acrylamide, les homo- et copolymères acryliques et les homo- et copolymères d'acide acrylamido méthylpropane sulfonique (AMPS).

Elle peut également contenir d'autres actifs que celui inclus dans les particules poreuses utilisées selon l'invention. Ces actifs additionnels peuvent être choisis parmi les agents desquamants, anti-microbiens, apaisants et sébo-régulateurs décrits précédemment.

Bien entendu, l'homme du métier veillera à choisir ce ou ces éventuels composés additionnels et/ou leur quantité de manière telle que les propriétés avantageuses de la composition selon l'invention ne soient pas, ou substantiellement pas, altérées par l'adjonction envisagée.

L'invention sera maintenant illustrée par l'exemple non limitatif suivant.

### Exemple : Fluide pour peaux grasses

### a) Préparation des particules poreuses chargées

0,5 g de tocophérol et 1 g d'acide n-octanoyl-5-salicylique sont solubilisés dans l'acétone. Dans ce mélange, on introduit 13,5 g de particules poreuses de Nylon-12 commercialisées sous la dénomination "ORGASOL 2002 UD NAT COS" par la société ATOFINA. La dispersion est ensuite introduite dans un évaporateur rotatif afin d'éliminer l'acétone. On obtient alors une poudre chargée en tocophérol et dérivé d'acide salicylique.

### b) Préparation de la composition cosmétique

On prépare une émulsion H/E ayant la composition ci-dessous, de façon classique pour l'homme du métier.

| | | |
|---|---|---|
| **A** - | Eau | 61 % |
| | Glycérine | 5 % |
| | Conservateurs | 0.5 % |
| **B** - | Stéarate de glycéryle et de polyoxyéthylène (100 OE) | 2 % |
| | Alcool cétylique | 1 % |
| | Polysorbate-20 | 2 % |
| | Conservateurs | 0.2 % |
| | Isononanoate d'isononyle | 6 % |
| | Octyldodécanol | 4 % |
| | Acide n-octanoyl-5-salicylique | 0.5 % |
| **C** - | Cyclohexasiloxane | 5 % |
| | Gomme de xanthane | 0.2 % |
| | Copolymère acrylique (Pemulen TR2 de NOVEON) | 0.45 % |
| **D** - | Eau Qsp | 100 % |
| | Soude Qsp | pH 5 |
| **E** - | Particules de Nylon chargées | 3 % |
| **G** - | Copolymère diméthacrylate d'éthylène glycol/ méthacrylate de lauryle* | 1 % |

| | | |
|---|---|---|
| *"POLYTRAP® 6603" commercialisé de la société RP SCHERER | | |

### Mode Opératoire

La phase A est chauffée sous agitation à 80°C jusqu'à parfaite solubilisation puis la température est ramenée à 70°C. La phase B est chauffée sous agitation à 70°C jusqu'à obtention d'une phase limpide puis ajoutée à la phase A sous agitation. La gomme de xanthane et le copolymère acrylique sont dispersés dans le cyclohexasiloxane à température ambiante pendant 10 minutes et la phase C est ajoutée au mélange A + B. Puis la phase D, préalablement solubilisée est ajoutée au mélange ainsi obtenu. Le mélange A+B+C+D est ensuite refroidi à 25°C. Les phases E et F sont successivement dispersées au mélange précédemment obtenu.

Cette composition peut être appliquée matin et/ou soir sur le visage pour matifier les peaux grasses à tendance acnéique.

## Revendications

1. Procédé cosmétique de soin des peaux grasses, comprenant l'application topique sur la peau d'une composition comprenant, dans un milieu physiologiquement acceptable adapté à une application topique sur la peau et renfermant une phase aqueuse : (a) des particules poreuses ayant un diamètre moyen, en volume, inférieur ou égal à 10 µm et renfermant au moins un actif de soin des peaux grasses, et (b) au moins un composé absorbant et/ou adsorbant le sébum ayant une taille moyenne de particule en nombre supérieure à 10 µm, l'actif de soin des peaux grasses étant choisi parmi l'acide cinnamique, l'acide n-octanoyl-5-salicylique, le 2,4,4'-trichloro-2'-hydroxy diphényl éther, l'acide undécylénique, l'acide lipoïque, l'acide azélaïque, l'acide arachidonique, l'iodopropynyl butylcarbamate, le farnesol, l'acide β-glycyrrhétinique, le stearyl glycyrrhétinate, l'acide 3- stéaroyloxy glycyrrhétique, l'acide ursolique, le bisabolol, le tocophérol, l'acétate de tocophérol.

2. Procédé selon la revendication 1, **caractérisé en ce que** les particules poreuses sont choisies parmi des particules de polyamide, des particules de polyméthacrylate de méthyle, des particules de silice et des particules d'hydroxyapatite.

3. Procédé selon la revendication 1 ou 2, **caractérisé en ce que** les particules poreuses sont des particules de polyamide.

4. Procédé selon l'une quelconque des revendications 1 à 3, **caractérisé en ce que** les particules poreuses ont un diamètre moyen en volume supérieur ou égal à 0,1 1 µm.

5. Procédé selon l'une quelconque des revendications 1 à 4, **caractérisé en ce que** la composition contient de 1 à 5% en poids de particules poreuses chargées d'actif, par rapport au poids total de la composition.

6. Procédé selon l'une quelconque des revendications précédentes, **caractérisé en ce que** l'actif de soin des peaux grasses est l'acide n-octanoyl-5-salicylique.

7. Procédé selon l'une quelconque des revendications 1 à 5, **caractérisé en ce que** l'actif de soin des peaux grasses est l'iodopropynyl butylcarbamate.

8. Procédé selon l'une quelconque des revendications 1 à 5, **caractérisé en ce que** l'actif de soin des peaux grasses est choisi parmi l'acide -glycyrrhétinique, l'alpha-bisabolol.

9. Procédé selon l'une quelconque des revendications 1 à 5, **caractérisé en ce que** l'actif de soin des peaux grasses est choisi parmi le tocophérol et l'acétate de tocophérol.

10. Procédé selon l'une quelconque des revendications précédentes, **caractérisé en ce que** l'actif représente de 5 à 30% du poids total des particules chargées.

11. Procédé selon l'une quelconque des revendications précédentes, **caractérisé en ce que** les particules poreuses renfermant l'actif sont susceptibles d'être obtenues selon un procédé d'imprégnation comprenant les étapes suivantes :
- la solubilisation de l'actif à encapsuler dans un solvant tel que l'acétone ou l'éthanol, pour obtenir une solution d'imprégnation,
- l'imprégnation des particules poreuses par ladite solution d'imprégnation,
- l'évaporation du solvant jusqu'à l'obtention d'une poudre sèche.

12. Procédé selon l'une quelconque des revendications précédentes, **caractérisé en ce que** le composé absorbant et/ou adsorbant le sébum est de nature organique.

13. Procédé selon la revendication précedente, **caractérisé en ce que** le composé absorbant et/ou adsorbant le sébum est choisi parmi les poudres de polyamide : les poudres de polyméthacrylate de méthyle/diméthacrylate d'éthylène glycol ; les poudres de polyméthacrylate d'allyle/diméthacrylate d'éthylène glycol ; les poudres de copolymère diméthacrylate d'éthylène glycol/méthacrylate de lauryle ; les poudres de polyalkylstyrène réticulé ; et leurs mélanges.

14. Procédé selon l'une quelconque des revendications précédentes, **caractérisé en ce que** le composé absorbant et/ou adsorbant le sébum représente de 1 à 5% en poids, par rapport au poids total de la composition.

## Claims

1. Cosmetic method of caring for greasy skin, comprising the topical application to the skin of a composition comprising, in a physiologically acceptable medium suitable for topical application to the skin and containing an aqueous phase: (a) porous particles having an average diameter by volume of less than or equal to 10 µm and containing at least one greasy-skin-care active and (b) at least one sebum-absorbing and/or sebum-adsorbing compound having a number-average particle size of more than 10 µm, the greasy-skin-care active being selected from cinnamic acid, n-octanoyl-5-salicylic acid, 2,4,4'-trichloro-2'-hydroxydiphenyl ether, undecylenic acid, lipoic acid, azelaic acid, arachidonic acid, iodopropynyl butylcarbamate, farnesol, β-glycyrrhetinic acid, stearyl glycyrrhetinate, 3-stearoyloxyglycyrrhetic acid, ursolic acid, bisabolol, tocopherol and tocopherol acetate.

2. Method according to Claim 1, **characterized in that** the porous particles are selected from polyamide particles, polymethyl methacrylate particles, silica particles and hydroxyapatite particles.

3. Method according to Claim 1 or 2, **characterized in that** the porous particles are polyamide particles.

4. Method according to any one of Claims 1 to 3, **characterized in that** the porous particles have a volume-average diameter of greater than or equal to 0.1 µm.

5. Method according to any one of Claims 1 to 4, **characterized in that** the composition contains from 1% to 5% by weight of active-loaded porous particles, relative to the total weight of the composition.

6. Method according to any one of the preceding claims, **characterized in that** the greasy-skin-care active is 5-(n-octanoyl)salicylic acid.

7. Method according to any one of Claims 1 to 5, **characterized in that** the greasy-skin-care active is iodopropynyl butylcarbamate.

8. Method according to any one of Claims 1 to 5, **characterized in that** the greasy-skin-care active is selected from β-glycyrrhetinic acid and alpha-bisabolol.

9. Method according to any one of Claims 1 to 5, **characterized in that** the greasy-skin-care active is selected from tocopherol and tocopherol acetate.

10. Method according to any one of the preceding claims, **characterized in that** the active represents from 5% to 30% of the total weight of the loaded particles.

11. Method according to any one of the preceding claims, **characterized in that** the porous particles containing the active are obtainable according to an impregnation process comprising the following steps:
- dissolving the active to be encapsulated in a solvent such as acetone or ethanol to give an impregnating solution,
- impregnating the porous particles with said impregnating solution,
- evaporating the solvent until a dry powder is obtained.

12. Method according to any one of the preceding claims, **characterized in that** the sebum-absorbing and/or sebum-adsorbing compound is organic in nature.

13. Method according to the preceding claim, **characterized in that** the sebum-absorbing and/or sebum-adsorbing compound is selected from powders of polyamide; powders of polymethyl methacrylate/ethylene glycol dimethacrylate; powders of polyallyl methacrylate/ethylene glycol dimethacrylate; powders of ethylene glycol dimethacrylate/lauryl methacrylate copolymer; powders of crosslinked polyalkylstyrene; and mixtures thereof.

14. Method according to any one of the preceding claims, **characterized in that** the sebum-absorbing and/or sebum-adsorbing compound represents from 1% to 5% by weight relative to the total weight of the composition.

## Patentansprüche

1. Kosmetisches Verfahren für die Pflege fettiger Haut, das das Aufbringen einer Zusammensetzung auf topischem Wege auf die Haut umfasst, die in einem physiologisch akzeptablen Medium, das für ein topisches Aufbringen auf die Haut geeignet ist und eine wässrige Phase aufweist, enthält: (a) poröse Partikel mit einem auf das Volumen bezogenen mittleren Durchmesser von höchstens 10 µm, die mindestens einen Wirkstoff für die Pflege fettiger Haut enthalten, und (b) mindestens eine Sebum absorbierende und/oder adsorbierende Verbindung mit einer zahlenmittleren Partikelgröße über 10 µm, wobei der Wirkstoff für die Pflege fettiger Haut unter Zimtsäure, 5-n-Octanoyl-salicylsäure, 2,4,4'-Trichlor-2'-hydroxy-diphenylether, Undecylensäure, Liponsäure, Azelainsäure, Arachidonsäure, Iodpropinylbutylcarbamat, Farnesol, β-Glycyrrhetinsäure, Stearylglycyrrhetinat, 3-Stearoyloxyglycyrrhetinsäure, Ursolsäure, Bisabolol, Tocopherol und Tocopherolacetat ausgewählt ist.

2. Verfahren nach Anspruch 1, **dadurch gekennzeichnet, dass** die porösen Partikel unter den Polyamidpartikeln, Polymethylmethacrylatpartikeln, Kieselsäurepartikeln und Hydroxyapatitpartikeln ausgewählt sind.

3. Verfahren nach Anspruch 1 oder 2, **dadurch gekennzeichnet, dass** es sich bei den porösen Partikeln um Polyamidpartikel handelt.

4. Verfahren nach einem der Ansprüche 1 bis 3, **dadurch gekennzeichnet, dass** die porösen Partikel einen auf das Volumen bezogenen mittleren Durchmesser von größer oder gleich 0,1 µm besitzen.

5. Verfahren nach einem der Ansprüche 1 bis 4, **dadurch gekennzeichnet, dass** die Zusammensetzung 1 bis 5 Gew.-% mit Wirkstoff beladene poröse Partikel, bezogen auf das Gesamtgewicht der Zusammensetzung, enthält.

6. Verfahren nach einem der vorhergehenden Ansprüche, **dadurch gekennzeichnet, dass** es sich bei dem Wirkstoff für die Pflege fettiger Haut um die 5-n-Octanoyl-salicylsäure handelt.

7. Verfahren nach einem der Ansprüche 1 bis 5, **dadurch gekennzeichnet, dass** der Wirkstoff für die Pflege fettiger Haut das Iodpropinylbutylcarbamat ist.

8. Verfahren nach einem der Ansprüche 1 bis 5, **dadurch gekennzeichnet, dass** der Wirkstoff für die Pflege fettiger Haut unter Glycyrrhetinsäure und α-Bisabolol ausgewählt ist.

9. Verfahren nach einem der Ansprüche 1 bis 5, **dadurch gekennzeichnet, dass** der Wirkstoff für die Pflege fettiger Haut unter Tocopherol und Tocopherolacetat ausgewählt ist.

10. Verfahren nach einem der vorhergehenden Ansprüche, **dadurch gekennzeichnet, dass** der Wirkstoff 5 bis 30 % des Gesamtgewichts der beladenen Partikel ausmacht.

11. Verfahren nach einem der vorhergehenden Ansprüche, **dadurch gekennzeichnet, dass** die porösen Partikel, die den Wirkstoff enthalten, nach einem Imprägnierverfahren mit den folgenden Schritten erhältlich sind:
- der einzukapselnde Wirkstoff wird in einem Lösemittel wie Aceton oder Ethanol gelöst, um die Imprägnierlösung zu bilden,
- die porösen Partikel werden mit der Imprägnierlösung getränkt,
- das Lösemittel wird verdampft, bis ein trockenes Pulver vorliegt.

12. Verfahren nach einem der vorhergehenden Ansprüche, **dadurch gekennzeichnet, dass** die Verbindung, die Sebum absorbiert und/oder adsorbiert, organischer Natur ist.

13. Verfahren nach dem vorhergehenden Anspruch, **dadurch gekennzeichnet, dass** die Verbindung, die Sebum absorbiert und/oder adsorbiert, unter Polyamidpulvern; Polymethylmethacrylat/ Ethylenglycoldimethacrylat-Pulvern; Polyallylmethacrylat/Ethylenglycomdimethacrylat-Pulvern; Pulvern des Ethylenglycoldimethacrylat/Laurylmethacrylat-Copolymers; Pulvern von vernetztem Polyalkylstyrol; und deren Gemischen ausgewählt ist.

14. Verfahren nach einem der vorhergehenden Ansprüche, **dadurch gekennzeichnet, dass** die Verbindung, die Sebum absorbiert und/oder adsorbiert, 1 bis 5 Gew.-%, bezogen auf das Gesamtgewicht der Zusammensetzung, ausmacht.
